# EUROPEAN PATENT SPECIFICATION

(11) **EP 4 243 738 B1**
(45) Date of publication and mention of the grant of the patent: **01.04.2026**
(21) Application number: 21810017.0
(22) Date of filing: 12.11.2021
(51) Int. Cl.: A61F 2/38, D04H 18/02, A61B 17/04

(54) **MEDICAL IMPLANT, METHOD FOR MANUFACTURING A MEDICAL IMPLANT, MEDICAL PRODUCT, ALIGNMENT TOOL, METHOD FOR CUSTOMIZING A MEDICAL IMPLANT, AND FELTING INSTRUMENT**
MEDIZINISCHES IMPLANTAT, VERFAHREN ZUR HERSTELLUNG EINES MEDIZINISCHEN IMPLANTATS, MEDIZINISCHES PRODUKT, AUSRICHTUNGSWERKZEUG, VERFAHREN ZUM ANPASSEN EINES MEDIZINISCHEN IMPLANTATS UND FILZINSTRUMENT
IMPLANT MÉDICAL, PROCÉDÉ DE FABRICATION D'UN IMPLANT MÉDICAL, PRODUIT MÉDICAL, OUTIL D'ALIGNEMENT, PROCÉDÉ DE PERSONNALISATION D'UN IMPLANT MÉDICAL, ET INSTRUMENT DE FEUTRAGE

(30) Priority: 12.11.2020 EP 20207151
(43) Date of publication of application: 20.09.2023
(73) Proprietor: ZuriMED Technologies AG, 8008 Zürich (CH)
(72) Inventor: BACHMANN, Elias, 8006 Zürich (CH); LI, Xiang, 8126 Zumikon (CH); MENGHINI, Danilo, 8006 Zurich (CH)
(74) Representative: Vossius & Partner Patentanwälte Rechtsanwälte mbB
(86) International application number: PCT/EP2021/081586
(87) International publication number: WO 2022/101445

(56) References cited:
- US-A- 4 919 667
- US-A1- 2008 021 554
- US-A1- 2010 152 530
- US-A1- 2017 296 323

## Description

The invention relates to a medical implant, particularly for meniscus repair or replacement, a method for manufacturing the medical implant, a medical product comprising the medical implant and a packaging, a kit including the medical implant with an alignment tool, a method for customizing a medical implant, and a kit including the medical implant with a surgical felting instrument.

Various medical implants to replace or repair defects in soft biological tissues, such as tendons, ligaments and cartilage, are know from the prior art. These are typically connected to parts of the body by sutures (in case of integration into soft tissues) or anchor structures (in case of integration into bone).

In general, although medical implants are designed to resemble biological structures that they replace, many implants substantially differ from the natural structures in their mechanical properties, which may lead to complications. Furthermore, the use of sutures and bone anchors to fix the medical implants to adjacent biological structures bears the danger of breakage or pullout, resulting in a need for repeated surgery.

For example, knee injuries are worldwide an issue in medicine and the number of operations related to the knee joints are increasing continuously. Almost 15% of the total cases are associated with loss of tissue of either one of the two menisci. The meniscus is a cartilaginous tissue placed in the knee that helps the stabilization and lubrication of it especially during tension and torsion of the joint. The problem of the loss of meniscal tissue forces the individual to a restriction of physical activities and results in a degeneration of the cartilage in the knee with the increasing of age. Low vascularization of the meniscal tissue combined with other factors, prevents the regeneration of the cells in the meniscal cartilage and leads to the nowadays solution of replacement of it with an artificial implant. The method of substituting the damaged part seems to diminish or avoid degeneration of the meniscus. Many different implants have already been developed or are currently in study.

Both approaches, total and partial replacement follow the meniscectomy which is a removal of meniscal tissue. When substituting only the damaged part, the fixation is performed with sutures that connects the implant to the intact healthy meniscus, whereas the total replacement is placed and kept in position through screws in the bones or simply by interlocking the implant between femur and tibia. The partial replacement preserves as much healthy natural meniscus as possible.

CN 204723455 U discloses a double-layered meniscus implant comprising a base layer from a PLGA fiber felt and a surface layer from a gelatin fiber felt.

CN 107669373 A describes an artificial meniscus implant from a fiber woven structure comprising interwoven radial and circumferential fibers.

US 2017/0202672 A1 discloses an artificial meniscus comprising a polycarbonate-urethane resin reinforced by an inert fiber fabric distributed throughout the resin.

US 2010/152530 A1 discloses tissue engineering devices for pelvic floor repair. More specifically, tissue engineering devices made of an implant, having a central portion at least partially embedded within a nonwoven felt.

US 2008/021554 A1 discloses a felt for repairing soft tissue defects comprising a membranous collagen substrate and a bioresorbable fiber felted onto the collagen substrate. Methods of preparing a felt and methods of repairing soft tissue damage with a felt are also provided.

US 2017/296323 A1 is directed to apparatuses and methods for fabricating tubular structures from a combination of fibrous materials for use in, for example, tissue engineering scaffold applications. These materials may also be useful in other biological or non-biological applications in which such tubular fibrous structures may be applicable, examples including conventional medical devices, filters, fiber optics, cable wraps, geotextiles, batteries, fuel cells, armor, and other diverse applications.

US 4,919,667 A discloses a soft tissue implant in the form of a meniscus cartilage replacement for patient. Appropriately shaped top and bottom layers sandwich therebetween at least one intermediate felted player.

Some core challenges of these and other meniscus implants according to the prior art, especially for the partial meniscus repair, concerns the suturing fixation, the surface match and the mimic of the mechanical properties in different zones. Suturing fixation might be demanding and intricated for the surgeon and furthermore the strength of the attachment depends on very few stitches and the entire load is spread on them. Non-optimal partial implants can cause surface or size disparities that has as a possible consequence a higher contact stress. This might increase the friction between the articulating cartilage and the meniscus. The mechanical properties of the native meniscus are not isotropic, instead, they vary in different zones to fulfil the needs of different load bearings. The artificial replacements mentioned above do not have controllable mechanical properties in different zones.

Thus, the objective of the present invention is to provide a medical implant, particularly for meniscus replacement or repair, which overcomes the above-stated disadvantages of the prior art, in particular a medical implant which more closely resembles the mechanical properties of the natural replaced or repaired tissue.

This objective is attained by the subject matter of the independent claims. Embodiments of the invention are specified in the dependent claims and described hereafter.

A first aspect of the invention relates to a medical implant, particularly for lateral or medial meniscus repair or replacement, wherein the medical implant extends along a plane perpendicular to a first axis, and wherein the medical implant comprises, particularly is at least partially formed from, a felt material comprising a plurality of fibers.

According to a first variant of the first aspect of the invention, a density of the fibers varies along the plane resulting in a varying compressive strength along said first axis. According to a second variant of the first aspect of the invention (which may be applied independently of the first variant or in combination with the first variant), a percentage of the fibers aligned in a circumferential direction in respect of the first axis differs along the plane of the implant resulting in a varying tensile strength of the medical implant along the plane, wherein the tensile strength is measured parallel to the plane, more particularly wherein the tensile strength is measured in the circumferential direction.

As used herein, the term "medical implant" describes a device suitable for being inserted into a human or animal body, particularly to replace or augment damaged parts of the body.

The medical implant extends along a plane. Of course, the implant does not have to be perfectly planar, i.e., the implant may be curved along the first and second coordinate. Furthermore, the implant may be deformable, particularly wherein the implant is planar in an undeformed state (without applying external forces). The first axis can be described as a surface normal of the plane, e.g., in case the plane extends along the width and length of the implant, the first axis would extend along the height. In case of a meniscus implant, said plane may extend essentially in the medial-to-lateral and anterior-to-posterior direction, and the first axis may extend parallel to the tibia bone when the medical implant is arranged in the natural location of the meniscus.

The density of fibers is defined as the number of fibers per unit of volume of the medical implant. In particular, the percentage of the fibers aligned in the circumferential direction relates to the sum of the length of each individual fiber extending in the circumferential direction divided by the total length. The term "compressive strength" as used herein is a physical parameter describing the amount of compressive deformation of the medical implant in the direction of the first axis per force applied in the direction of the first axis. The term "tensile strength" as used herein is a physical parameter describing amount of stretching deformation of the medical implant in a direction parallel to the plane, particularly in the circumferential direction, per force applied in the direction parallel to the plane, particularly in the circumferential direction.

According to the invention, the density of the fibers and/or the percentage of fibers aligned in the circumferential direction varies along the plane, meaning the density and/or percentage is inhomogeneous in at least one direction along the plane, particularly resulting in a corresponding inhomogeneous distribution of compressive strength and/or tensile strength. In particular, this compressive/tensile strength distribution resembles the compressive/tensile strength distribution of the natural lateral or medial meniscus, resulting in improved function of the implant. The inhomogeneous distribution of fiber density and/or fiber alignment, and the corresponding compressive/tensile strength distribution may be characterized by different zones divided by a stepwise increase or decrease of density and/or fiber alignment or characterized by a gradual increase or decrease of density and/or fiber alignment.

The areas with increased density of the fibers may also result in a locally decreased thickness of the medical implant along the first axis. Therefore, in particular, the 3-dimensional structure of the implant is controllable by tuning the density of the fibers.

Due to the felt material, the 3D structure and mechanical properties of the medical implant may be easily adapted to the needs of the medical application and individual patient: To this end, for example, a felting needle comprising at least one barb may be advanced through the felt material at specific locations and in a specific direction to achieve certain densities and fiber alignments. E.g., by felting in a specified direction, the fibers can be preferentially aligned along this direction.

An additional advantage of the felt material is that it may be tightly connected to biological soft tissue, such as tendon, ligament or cartilage, by placing the implant on the tissue and repeatedly advancing a surgical felting needle comprising at least one barb through the felt material and the tissue resulting in fibers of the felt material being introduced into the tissue to achieve a mechanically strong suture-less fixation that spreads the anchorage on many different spots.

In certain embodiments, the medical implant comprises an outside surface, wherein the density of the fibers and/or the percentage of the fibers aligned in the circumferential direction increases in a radial direction in respect of the first axis towards the outside surface, particularly such that the compressive strength of the medical implant measured parallel to the first axis and/or the tensile strength of the medical implant measured parallel to the plane, more particularly in the circumferential direction, increases in the radial direction towards the outside surface. The outside surface particularly extends in a circumferential direction in respect of the first axis. In particular, in case of a meniscus implant, the outside surface may extend circumferentially to the first axis from the anterior side via the medial side or lateral side to the posterior side.

This distribution of compressive strength and/or tensile strength resembles the mechanical properties of the natural meniscus (e.g., a similar load distribution as that of the natural meniscus) and thus improves the function of the medical implant.

In certain embodiments, the medical implant comprises a plurality of zones, wherein the density of the fibers and/or the percentage of the fibers aligned in the circumferential direction varies between the zones, wherein particularly the zones are arranged along the radial direction. In certain embodiments, the medical implant extends from a first end via a central section towards a second end along a curved line extending in a circumferential direction in respect of the first axis, wherein the medical implant comprises a central recess, around which the curved line extends. In other words, the medical implant is C-shaped or crescent moon shaped, particularly resembling the shape of the natural lateral or medial meniscus.

In certain embodiments, the plurality of zones comprises a first zone, a second zone, a third zone and/or a fourth zone.

In certain embodiments, the zones each extend in the plane between the first end and the second end along the curved line. In certain embodiments, the first zone is arranged along the edge of the central recess on the inside of the medical implant. In certain embodiments, the second zone is arranged outside of the first zone along the radial direction, and the third zone is arranged outside of the second zone along an outside surface of the medical implant. In certain embodiments, the fourth zone is arranged around the entire outside surface, wherein particularly the fourth zone is formed by the first cover layer and/or the second cover layer.

In certain embodiments, the density of the fibers increases from the first zone to the second zone, and from the second zone to the third zone. The varying compressive strength in the plane particularly resembles the mechanical properties of the natural meniscus.

In certain embodiments, the percentage of the fibers aligned in the circumferential direction differ between the first zone, the second zone, the third zone and/or the fourth zone.

In certain embodiments, the percentage of fibers aligned in the circumferential direction increases from the first zone towards the second zone, and from the second zone towards the third zone, and then decreases from the third zone to the fourth zone.

In certain embodiments, the percentage of fibers aligned in the circumferential direction is less than 60 % in the first zone, 30% to 80 % in the second zone, more than 50 % in the third zone, and less than 60 % in the fourth zone.

According to certain embodiments, the tensile strength of the medical implant in the plane P, particularly in the circumferential direction C, is in the range of 0,050 MPa to 90 MPa in the first zone, 0,750 MPa to 140 MPa in the second zone, 0,100 MPa to 176 MPa in the third zone, and 0,050 MPa to 16 MPa in the fourth zone.

In certain embodiments, the medical implant is formed from a plurality of layers comprising the felt material, wherein the layers are stacked in a direction parallel to the first axis, and wherein the layers are connected by felting. Therein, "felting" refers to advancing a felting needle comprising at least one barb repeatedly through at least two adjacent stacked layers resulting in fibers of one layer extending into the other layer to connect the layers.

Stacking layers comprising a felt material allows a simple and versatile manufacturing method for the medical implant.

In certain embodiments, the density of the fibers of at least one layer of the plurality of layers, particularly the density of the fibers of each layer of the plurality of layers, varies along the plane, particularly resulting in a varying compressive strength of the respective layer along the first axis.

In certain embodiments, the percentage of the fibers aligned in the circumferential direction of at least one layer of the plurality of layers, particularly the percentage of the fibers aligned in the circumferential direction of the fibers of each layer of the plurality of layers, varies along the plane, particularly resulting in a varying tensile strength of the respective layer along the plane, particularly in the circumferential direction.

In certain embodiments, at least one layer of the plurality of layers, particularly each layer of the plurality of layers, comprises an outside edge, wherein the density of the fibers and/or the percentage of the fibers aligned in the circumferential direction increases in a radial direction in respect of the first axis towards the outside edge, particularly such that the compressive strength of the respective layer measured parallel to the first axis and/or the tensile strength of the respective layer measured parallel to the plane, more particularly in the circumferential direction, increases in the radial direction towards the outside edge. The outside edge particularly extends in a circumferential direction in respect of the first axis. In particular, in case of a meniscus implant, the outside edges in the implant assembled from the layers may extend circumferentially to the first axis from the anterior side via the medial side or lateral side to the posterior side.

In certain embodiments, at least one layer of the plurality of layers, particularly each layer of the plurality of layers, comprises a plurality of zones, wherein the density of the fibers and/or the percentage of the fibers aligned in the circumferential direction varies between the zones, wherein particularly the zones are arranged along the radial direction.

By cutting the above-described layers into a desired shape and connecting the layers by felting, a medical implant having a desired 3D-distribution of mechanical properties can be easily manufactured.

In certain embodiments, the layers comprise a bottom layer and a top layer, wherein an arc length of the bottom layer extending along the curved line is greater than an arc length of the top layer extending along the curved line, such that a thickness of the medical implant measured parallel to the first axis increases, particularly stepwise, from the first end towards the central section and/or from the second end towards the central section. In case of a stepwise thickness increase, these steps may be smoothed by providing a cover layer that covers the bottom layer and the top layer. The bottom and top layers comprising the felt material may be connected, for instance, by felting.

In certain embodiments, the layers comprise at least one intermediate layer between the bottom layer and the top layer, wherein an arc length of the at least one intermediate layer is between the arc length of the bottom layer and the arc length of the top layer.

In this manner, a desired 3D-architecture of the implant comprises different steps in thickness and accordingly a desired steepness can be achieved in an easy manner. The bottom, top and intermediate layers comprising the felt material may be connected, for instance, by felting.

In certain embodiments, the medical implant comprises a first cover layer covering the bottom layer and the top layer, and particularly the at least one intermediate layer, on a top surface extending along the plane of the medical implant, particularly from the first end towards the second end. This advantageously results in smoothing of a stepwise thickness increase mediated by the different layers.

In certain embodiments, the medical implant comprises a second cover layer covering the bottom layer and the top layer, and particularly the at least one intermediate layer on a rim surface extending parallel to the first axis and circumferentially in respect of the first axis. This particularly provides a smooth lateral rim surface.

In certain embodiments, the medical implant comprises a strip comprising the felt material, wherein the strip is arranged circumferentially in respect of the first axis along an outside surface of the medical implant between the bottom layer and the top layer.

In certain embodiments, the strip extends along the curved line.

In certain embodiments, the strip has an arc length along the curved line which is shorter than the arc length of the bottom layer and shorter than the arc length of the top layer.

In certain embodiments, the medical implant comprises a replacement member configured to replace a part of a soft biological tissue, particularly a meniscus, and a supporting body connected to the replacement member, wherein the supporting body extends from the first end via the central section to the second end along the curved line in the circumferential direction, and wherein the replacement member is arranged on a bottom surface of the supporting body (arranged parallel to the plane), wherein the replacement member covers a partial segment of the bottom surface. In particular, the supporting body comprises a central recess, around which the curved line extends. In other words, the supporting body is C-shaped or crescent moon shaped, resembling the shape of the natural lateral or medial meniscus.

In certain embodiments, the supporting body and/or the replacement member is formed by a plurality of layers comprising a felt material.

In certain embodiments, the replacement member has a thickness measured parallel to the first axis which is greater than a thickness of the supporting body measured parallel to the first axis.

In case of a meniscus implant, the replacement member may be inserted in a gap in the natural meniscus obtained by surgical removal of a part of the meniscus, and the supporting body may be placed on top of the natural meniscus to mechanically stabilize the replacement member.

In certain embodiments, the medical implant, particularly the felt material, comprises or consists of polyethylene terephthalate (PET), polytetrafluoroethylene (PTFE), polyethylene (PE), poly(lactic-co-glycolic acid) fiber felt, biometric collagen, silk fibers, polycarbonate polyurethane (PCU) fibers, or polycaprolactone (PCL).

A second aspect of the invention relates to a method of manufacturing a medical implant according to the first aspect, wherein at least one sheet comprising a felt material is provided, the sheet extending along a plane perpendicular to a first axis, wherein the felt material comprises a plurality of fibers, and wherein a felting needle comprising at least one barb is repeatedly advanced through the sheet at specified locations to increase the density of the fibers such that a density of the fibers varies along the plane and/or such that a percentage of the fibers aligned in a circumferential direction in respect of the first axis differs along the plane, such that the compressive strength of the sheet measured along the first axis is increased at the specified locations, and/or to increase the percentage of fibers aligned in a circumferential direction in respect of the first axis at the specified locations, such that the tensile strength of the sheet measured along the plane, more particularly in the circumferential direction, is increased at the specified locations.

The barb or barbs of the felting needle pull fibers of the felt material in the direction of movement of the needle, and therefore increase the percentage of fibers aligned along this direction. In addition, particularly due to entanglement of fibers caused by the felting needle, the density of the fibers along the first axis is increased, which may result in a decreased thickness (in the direction of felting) of the felt material at the location where the felting needle is introduced.

In certain embodiments of the method of manufacturing, a plurality of layers comprising a felt material are provided, wherein the layers are connected by repeatedly advancing a felting needle comprising at least one barb through the layers to connect the layers.

In certain embodiments, the layers are assembled from a raw or processed felt material comprising fibers.

In certain embodiments, the layers are formed by electrospinning.

In certain embodiments, the layers are formed by 3D printing.

A third aspect of the invention relates to a medical product comprising a medical implant according to the first aspect, and a transparent packaging, wherein the packaging comprises printed markings comprising an outline marking of the shape of the medical implant, a radial scale marking and a circumferential scale marking, wherein the medical implant is arranged in the packaging, such that the outline marking is aligned with the medical implant. The packaging may be, e.g., a pouch or a blister. In particular, the medical implant is sealed in the packaging, more particularly in an airtight manner.

In case of a meniscus implant, the outline marking may extend along a curved line extending in a circumferential direction in respect of the first axis around a central recess. In other words, the outline marking is C-shaped or crescent moon shaped, resembling the shape of the natural lateral or medial meniscus.

A fourth aspect of the invention relates to a kit including the medical implant according to the first aspect and/or the medical product according to the third aspect with an alignment tool comprising a working surface configured to receive the medical implant according to the first aspect and/or the medical product according to the third aspect. The alignment tool further comprises a holder configured to be placed on the working surface, wherein the holder comprises a cutout having a size and shape of the medical implant, such that the outline marking and the medical implant can be aligned with the cutout.

This alignment tool particularly provides a working surface for accurate alignment and connection by a felting needle of different components of the medical implant, e.g. of the above-described layers (such as the top layer, the bottom layer and the at least one intermediate layer). The alignment tool particularly aids in connecting a supporting body to a replacement member, thereby generating a two-part implant for partial meniscus replacement. In certain embodiments, the holder of the alignment tool comprises a plurality of notches arranged around an edge of the cutout, wherein the notches extend in the radial direction and/or in the circumferential direction.

Such notches facilitate cutting with a scalpel along the radial direction to cut a medical implant placed on the working surface into a desired shape.

In certain embodiments, the alignment tool comprises at least one fixing means configured to fix the holder to the working surface, particularly to clamp the medical product between the working surface and the holder.

In certain embodiments, the working surface is comprised in a replaceable part of the alignment tool.

In certain embodiments, the working surface is smooth.

In certain embodiments, the working surface is covered by a spongy material.

A smooth or a spongy surface may be advantageous for felting on the working surface, e.g. to avoid damage from the needle to the working surface by the spongy material.

A fifth aspect of the invention relates to a method for customizing a medical implant, particularly according to the first aspect, wherein a medical product according to the second aspect is provided, and wherein a desired shape of the medical implant to be customized is marked on the transparent packaging of the medical product using the radial scale marking and the circumferential scale marking, and wherein a piece is cut out of the medical implant and the packaging using the marked desired shape.

In certain embodiments, to obtain a medical implant custom-fit for partial meniscus replacement, a desired depth of the medical implant from the central recess outwards along the radial scale marking and a start coordinate defined by the distance to a root of the outline marking along the circumferential scale marking (the root being defined by a location of an open side of the central recess) are determined and a first line extending from the central recess along the radial scale marking by the determined depth is marked on the packaging, e.g. with a pen, at the start coordinate. Next, in particular, an end coordinate of the desired implant along the circumferential scale marking is determined and a second line is drawn on the packaging along the circumferential scale marking from the start coordinate to the end coordinate. Finally, in particular, a third line is drawn on the packaging from the end coordinate along the radial scale marking inside towards the edge of the central recess. In particular, the medical implant having the desired shape is subsequently cut out along the marked desired shape on the packaging, particularly using scissors or a scalpel.

In particular, this provides an easy and accurate method of generating a custom-made implant for partial meniscus replacement.

In certain embodiments, a supporting body comprising a felt material is arranged on the working surface of the alignment tool, wherein the medical product (which comprises a hole generated by cutting out the piece of the medical implant) and the packaging are arranged on the supporting body, and wherein the piece cut out of the medical implant is arranged in the hole of the packaging, and wherein a felting needle comprising at least one barb, particularly the surgical felting needle according to the sixth aspect, is repeatedly advanced through the piece and the supporting body, thereby connecting the piece to the supporting body by felting. In particular, the piece serves as a replacement member for a soft biological tissue, such as during partial meniscus replacement.

The alignment tool particularly aids in connecting a supporting body to a replacement member, thereby generating a two-part implant for partial meniscus replacement.

A sixth aspect of the invention relates to a kit including the medical implant according to the first aspect and/or the medical product according to the third aspect with a felting instrument, particularly a surgical felting instrument, comprising a felting needle, the felting needle comprising at least one barb, wherein the felting instrument comprises a drive configured to move the felting needle back and forth along a longitudinal axis.

By the at least one barb of the felting needle, the fibers of a felt material are pushed and/or pulled when the needle is advanced through the felt material. Thereby, it is possible to connect different pieces of felt material, connect felt material to biological tissue, and tune the mechanical and structural properties of the medical implant by increasing the density of the fibers and altering the fiber alignment as described above.

In particular, the felting needle comprises a plurality of barbs, more particularly at least one forward barb extending towards the tip of the felting needle and configured to push fibers when the needle moves forward into the felt material and pull fibers when the needle moves in the reverse direction away from the felt material, and at least one reverse barb extending in the opposite direction away from the tip of the felting needle to pull fibers when the needle moves forward into the felt material and push fibers when the needle moves in the opposite direction away from the felt material.

In certain embodiments, the felting needle is curved.

The curved design in the needle results in a reduced penetration depth into tissue, which avoids the tip of the needle touching and potentially damaging a bone (e.g. in case of connecting a medical implant to a soft tissue, such as a meniscus, in the vicinity of a bone, or to avoid the tip of the needle touching and potentially damaging a working surface (e.g., of the alignment tool), e.g., during connection of components (such as biological soft tissue)of the medical implant.

In certain embodiments, the felting instrument comprises a supporting member configured to hold a medical implant comprising a felt material comprising a plurality of fibers, particularly a medical implant according to the first aspect, in place on a biological soft tissue, particularly a meniscus, such that the felting needle of the felting instrument can be advanced through the medical implant and the soft tissue to connect the medical implant to the biological soft tissue.

The supporting member avoids displacement of the medical implant relative to the soft biological tissue (e.g. a meniscus) during connection of the medical implant to the biological tissue by felting, which help to keep the implant stable and improves the performance of the surgeon. Moreover, the supporting member particularly avoids that the felting needle touches and potentially damages a bone in the vicinity of the soft tissue.

In certain embodiments, the supporting member comprises a lower member configured to be arranged below the soft biological tissue, particularly between the soft biological tissue and a bone, and/or through the soft biological tissue.

In certain embodiments, the lower member comprises at least one needle comprising a tip configured to penetrate the soft biological tissue.

For example, during meniscus repair or replacement, the lower member may be advanced between the tibia bone and the natural meniscus. Optionally, in case the lower member comprises a needle with a sharp tip, the lower member may be advanced at least partially through the natural meniscus.

In certain embodiments, the supporting member comprises an upper member configured to be arranged on the medical implant, particularly to apply pressure on the medical implant.

By means of the upper member, it is possible to apply pressure on the medical implant placed on the biological soft tissue, e.g. the meniscus to hold the implant in place.

In certain embodiments, the lower member comprises an end section that is flippable to connect the end section to the upper member. In particular, a cage-like compartment holding the assembly between the medical implant and the soft biological tissue is thereby formed. The cage like-compartment provides further stabilization of the implant on the tissue and provides a protected working environment for felting.

In certain embodiments, the supporting member is spaced apart from the felting needle of the felting instrument, such that contact between the felting needle and the supporting member is avoided when the felting needle moves back and forth along the longitudinal axis.

The disclosure also describes a surgical method for connecting a medical implant, particularly according to the first aspect, to soft biological tissue, wherein a felting instrument according to the sixth aspect is provided, wherein a medical implant comprising a felt material comprising fibers is arranged on a soft biological tissue, particularly on a ligament, a tendon or cartilage, more particularly on a medial or lateral meniscus, and wherein the felting needle of the felting instrument is repeatedly advanced through the medical implant and the soft biological tissue to connect the medical implant to the soft biological tissue.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.
- Fig. 1: depicts a sheet from a felt material and a felting instrument comprising a felting needle for changing the density and alignment of fibers in the felt material;
- Fig. 2: shows embodiments of a medical implant according to the invention for meniscus repair or replacement;
- Fig. 3: shows the fiber alignment in different zones of a medical implant according to the invention;
- Fig. 4: shows the assembly of a medical implant according to an embodiment of the invention from layers comprising a felt material;
- Fig. 5: shows the assembly of a medical implant according to a further embodiment of the invention from layers comprising a felt material;
- Fig. 6: shows medical implants according to the invention comprising a replacement member and a supporting body;
- Fig. 7: shows a medical product according to the invention comprising a medical implant according to the invention arranged in a transparent packaging comprising markings;
- Fig. 8: illustrates a method for obtaining a medical implant of a desired shape from the medical product shown in Fig. 7;
- Fig. 9: shows an alignment tool according to the invention;
- Fig. 10-11: show a method for obtaining a medical implant according to the invention comprising a replacement part and a supporting body using the medical product shown in Fig. 7 and 8 and the alignment tool shown in Fig. 9;
- Fig. 12: depicts a method for connecting fibers of a felt material to a soft tissue using a felting instrument according to the invention;
- Fig. 13: shows an embodiment of the felting instrument according to the invention used for connecting a medical implant according to the invention to soft tissue;
- Fig. 14: shows a further embodiment of the felting instrument according to the invention used for connecting a medical implant according to the invention to soft tissue;
- Fig. 15: shows an embodiment of the felting instrument according to the invention.

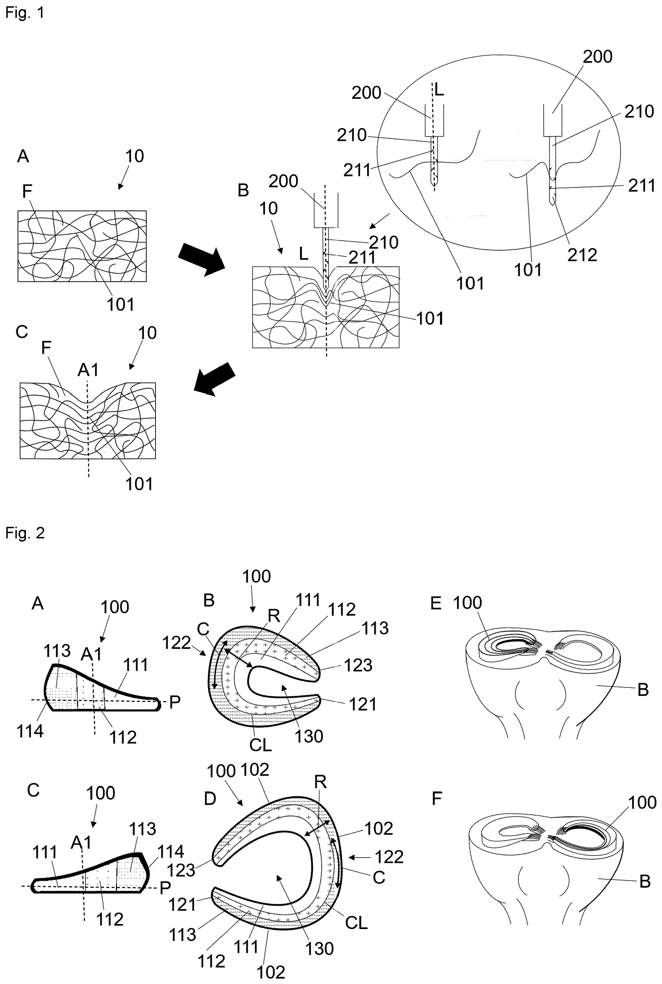

Fig. 1A schematically depicts a sheet 10 from a felt material F comprising a plurality of randomly arranged fibers 101.

As part of a manufacturing method for a medical implant 100 according to the invention, a felting needle 210 of a felting instrument 200, the felting needle 210 comprising a plurality of barbs 211, may be advanced with its tip 212 into the sheet 10 and repeatedly moved back and forth along a longitudinal axis L, which in this case extends along the felting needle 210 (Fig. 1B, details of the felting instrument 200 shown in the inset of Fig. 1B).

Thereby, the barbs 211 of the felting needle 210 pull and push fibers 101 of the felt material F, which results in a changed density of the fibers 101 and in an altered alignment of the fibers 101 (Fig. 1C). In the depicted example, the density of the sheet 10 is increased at the position where the felting needle 210 has been introduced, resulting in a denser packing of the fibers 101 and therefore a decreased thickness along the first axis A1.

Fig. 2A-D show embodiments of a medical implant 100 comprising a felt material F for meniscus replacement according to the invention. Fig. 2A and 2B show medical implants 100 designed to replace the lateral meniscus, and Fig. 2C and 2D depict medical implants 100 configured to replace the medial meniscus. Therein, Fig. 2A and 2C are side views of the respective medical implant 100, and Fig. 2B and 2D are sectional views along a plane P perpendicular to the first axis A1 indicated in Fig. 2A and 2C. Fig. 2E shows the location of the medical implant 100 on a tibia bone B when replacing the lateral meniscus, and Fig. 2F shows the location of the medical implant 100 on a tibia bone when replacing the medial meniscus.

As best seen in Fig. 2B and 2D, the medical implant 100 extends along the plane P from a first end 121 via a central section 122 to a second end 123 along a curved line CL extended in a circumferential direction C in respect of the first axis A1. The medical implant 100 has a central recess 130 resulting in a C-shape or crescent moon shape which resembles the natural meniscus. The shape and size of the medical implant 100 differs slightly between the lateral meniscus implant (Fig. 2B) and the medial meniscus implant (Fig. 2D). E.g., in the medial meniscus implant, the central recess 130 is larger than in the lateral meniscus implant.

In the cross-sectional view of Fig. 2B and 2D, three different zones 111, 112, 113 differing in their mechanical properties are visible. The zones 111, 112, 113 each extend in the plane P between the first end 121 and the second end 123 along the curved line CL. The first zone 111 is arranged along the edge of the central recess 130 on the inside of the medical implant 100. The second zone 112 is arranged outside of the first zone 111 in a radial direction R, and the third zone 113 is arranged outside of the second zone 112 along an outside surface 102 of the medical implant 100. A fourth zone 114 (depicted by the thick line marking the outline of the medical implant 100 in Fig. 2A and 2C) is arranged around the entire outside surface 102.

In particular, the density of the fibers 101 of the felt material F in the medical implant 100 differ between the zones 111, 112, 113, 114 resulting in a varying compressive strength along the first axis A1 in the different zones 111, 112, 113, 114. More particularly, the density of the fibers 101 may increase from the first zone 111 to the second zone 112, and from the second zone 112 to the third zone 113. The varying compressive strength in the plane P particularly resembles the mechanical properties of the natural meniscus.

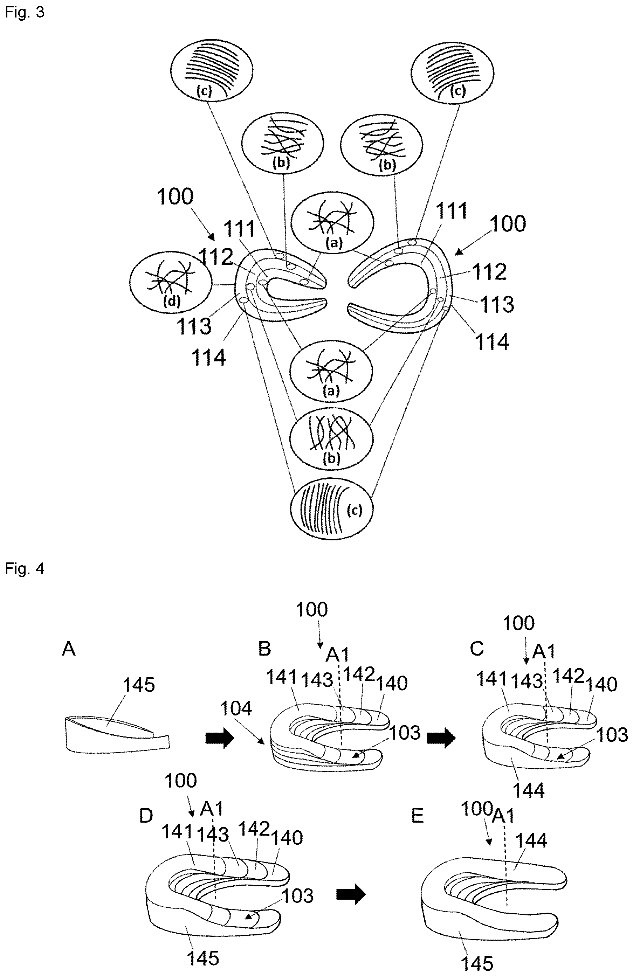

Furthermore, as illustrated in Fig. 3, the percentage of the fibers 101 aligned in the circumferential direction (see Fig. 2B and 2D) may differ between the zones 111, 112, 113, 114. Fig. 3 shows the lateral meniscus implant 100 (left) and the medial meniscus implant 100 (right) according to the invention comprising the zones shown in Fig. 2 and described above.

Two spots per first, second and third zone 111, 112, 113 and one spot of the fourth zone 114 are enlarged and the corresponding fiber alignment is illustrated in the insets (wherein (a) marks the first zone 111, (b) marks the second zone 112, (c) marks the third zone 113, an (d) marks the fourth zone 114).

As shown in Fig. 3, the percentage of fibers 101 aligned in the circumferential direction increases from the first zone 111 towards the second zone 112, and from the second zone 112 towards the third zone 113, and then decreases from the third zone 113 to the fourth zone 114. In the depicted example, the distribution of the fiber 101 directions appears almost random in the first zone 111 and the fourth zone 114, whereas a higher percentage of fibers 101 are oriented parallel to each other (and aligned in the circumferential direction) in the second zone 112, and almost all fibers 101 are parallel (and aligned in the circumferential direction) in the third zone 113.

According to certain embodiments, the percentage of fibers 101 aligned in the circumferential direction is less than 60 % in the first zone 111, 30% to 80 % in the second zone 112, more than 50 % in the third zone 113, and less than 60 % in the fourth zone 114.

The depicted alignment of fibers 101 in the circumferential direction C results in varying tensile strength of the medical implant in the different zones 111, 112, 113, 114, similar to the natural mechanical properties of the meniscus.

According to certain embodiments, the tensile strength of the medical implant in the plane P, particularly in the circumferential direction C, is in the range of 0,050 MPa to 90 MPa in the first zone 111, in the range of 0,750 MPa to 140 MPa in the second zone 112, in the range of 0,100 MPa to 176 MPa in the third zone 113, and in the range of 0,050 MPa to 16 MPa in the fourth zone 114.

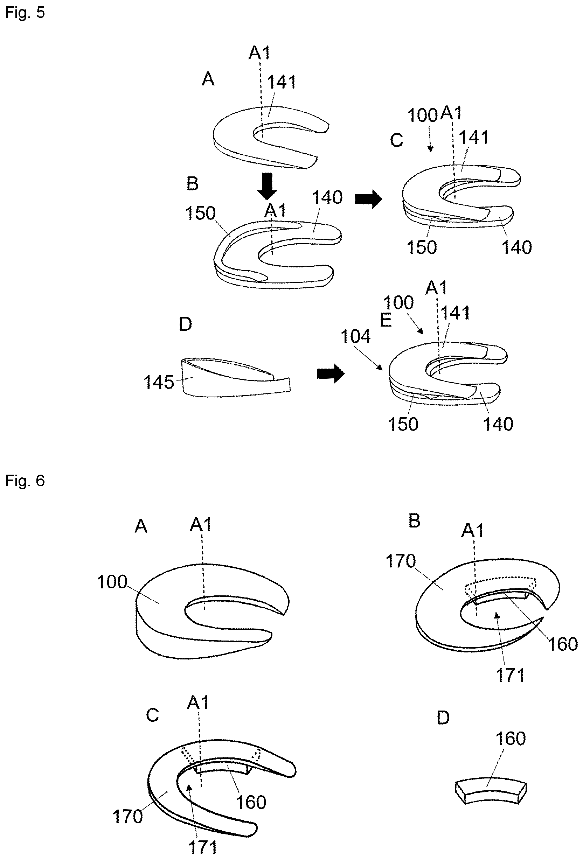

Fig. 4 and 5 depict two different methods of assembling a medical implant 100 from a felt material F for meniscus replacement according to the invention.

According to a first embodiment, as shown in Fig. 4, layers 140, 141, 142, 143 from a felt material F are stacked along the first axis A1 and connected to each other by advancing a felting needle 210 of a felting instrument 200 (see Fig. 1B) repeatedly through the stacked layers 140, 141, 142, 143 to felt the layers 140, 141, 142, 143 together. Therein, a bottom layer 140 is provided, and a first intermediate layer 142, a second intermediate layer 143 and a top layer 141 are subsequently stacked on top of the bottom layer 140 and then connected (either in separate steps to connect adjacent layers or in a single step to connect all layers simultaneously) by felting. The first intermediate layer 142 has a shorter arc length along the curved line CL depicted in Fig. 2 than the bottom layer 140, the second intermediate layer 143 has a shorter arc length along the curved line CL shown in Fig. 2, and the second intermediate layer 143 has a shorter arc length along the curved line CL than the top layer 141, resulting in a slope from the first end 121 to the central section 122 and from the second end 123 to the central section 122 (see reference signs indicated in Fig. 2). This results in a stepwise increase of thickness along the first axis A1 from the first end 121 to the central section 122 and from the second end 123 to the central section 122.

After assembling the layers 140, 141, 142, 143, a first cover layer 144 is arranged on a top surface 103 of the medical implant 100 and connected by felting, resulting in the arrangement shown in Fig. 4E. The first cover layer 144 equilibrates the steps formed at the interfaces between the adjacent layers 140, 141, 142, 143 and leads to a gradual thickness increase from the first and second end 121, 123 to the central section 122.

In addition, a second cover layer 145 (shown individually in Fig. 4A) is arranged on a rim surface 104 of the stacked layers 140, 141, 142, 143 (see Fig. 4B) and connected by felting (see Fig. 4C).

The first cover layer 144 and the second cover layer 145 may together constitute the fourth zone 114 as shown in Fig. 2 and 3.

The medical implant 100 depicted in Fig. 4 may be used e.g. for complete meniscus replacement after surgical removal of the natural lateral or medial meniscus.

Fig. 5 shows an assembly method of a further embodiment of the medical implant 100 according to the invention.

As shown in Fig. 5B, a strip 150 comprising a felt material F is first arranged along the outside edge of a bottom layer 140 comprising a felt material F. The strip 150 is then connected to the bottom layer 140 by felting. Subsequently, a top layer 141 from a felt material is arranged on top of the bottom layer 140 and the strip 150, and the components are connected by felting. Subsequently, a second cover layer is arranged on a rim surface 104 of the stacked bottom layer 140, strip 150 and top layer 141 and connected by felting.

Thereby, a gradual thickness increase from the first and second end 121, 123 to the central section 122 (see Fig. 2) is achieved.

The medical implant 100 depicted in Fig. 5 may be used e.g. for complete meniscus replacement after surgical removal of the natural lateral or medial meniscus.

In Fig. 6, a single piece medical implant 100 for meniscus replacement (Fig. 6A), and a two-piece medical implant 100 for partial meniscus replacement (resulting in meniscus repair, Fig. 6B and 6C) is shown. The medical implant 100 for partial meniscus replacement (Fig. 6B and 6C) comprises a replacement member 160 and a supporting body 170, both comprising a felt material F, wherein the replacement member 160 is connected to a bottom surface 171 of the supporting body 170 by felting. The replacement member 160 (shown individually in Fig. 6D) is configured to be inserted in a gap of the natural meniscus obtained by surgical removal of a part of the cartilage material of the natural meniscus to fill the gap. The supporting body 170 is configured to cover the remaining natural meniscus to mechanically support the replacement member 160. In particular, the supporting body 170 may be felted to the natural meniscus by a surgical felting instrument 200.

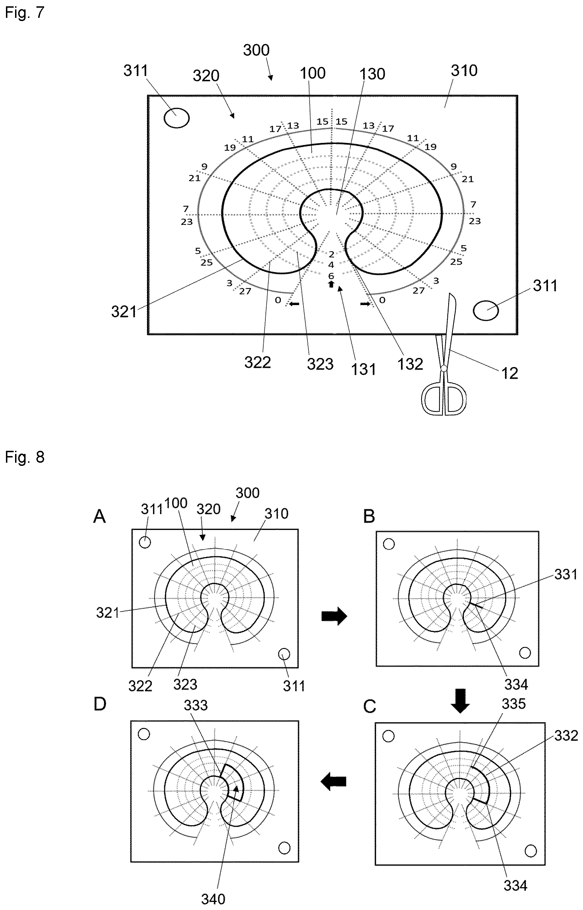

Fig. 7 shows a medical product 300 comprising a medical implant 100 for meniscus repair or replacement arranged in a transparent packaging 310. The transparent packaging 310 comprises printed markings 320, namely an outline marking 321, a radial scale marking 322 and a circumferential scale marking 323.

The outline marking 321 resembles the size and shape of the medical implant 100 in the packaging 310 and the medical implant 100 is arranged in the packaging 310, such that it is aligned with the outline marking 321.

The radial scale markings 322 consist of lines (e.g. dotted lines as shown in Fig. 7, or solid lines) extending in a radial direction R in respect of the first axis A1 of the medical implant 100 (see Fig. 2), wherein the lines point to the center of the central recess 130.

The circumferential scale markings 323 consist of lines (e.g. dotted lines as shown in Fig. 7, or solid lines) oriented in the circumferential direction C in respect of the first axis A1 of the medical implant 100 (see Fig. 2) along the medical implant 100.

By the crossing lines of the radial scale markings 322 and circumferential scale markings 323, a coordinate system is defined on the surface of the medical implant 100 in the packaging 310.

Radial coordinates from 2 to 6 indicating a radial distance from the edge of the central recess 130 are indicated at the opening of the central recess 130.

In a similar manner, circumferential coordinates ranging from 0 to 27 (0 being a root at a radial edge 132 of an opening 131 of the central recess 130) are indicated along the periphery of the medical implant in both clockwise and counterclockwise directions.

The packaging 310 further comprises two through-holes 311 for aligning the medical product 300 on an alignment tool 400 (see below).

The markings 320 can be used to cut out a piece 11 of a pre-selected size and shape from the medical implant 100, using scissors 12 or alternatively a scalpel, particularly to form a custom-sized replacement part 160 of a two-part medical implant 100 as shown in Fig. 6B and 6C.

Part of this process is illustrated in Fig. 8.

After an empty space has been created in the natural meniscus by partial meniscectomy, the position and size of the empty space is measured.

Then, as shown in Fig. 8B a first line 331 is drawn (using a pen) on the packaging 310 in the radial direction R with the help of the radial scale markings 322 and circumferential scale markings 323 according to the measured radial dimensions (depth) and start coordinate 334 in the circumferential direction C of the empty space of the natural meniscus.

Next, as shown in Fig. 8C, a second line 332 is drawn on the packaging 310 in the circumferential direction C between the start coordinate 334 and an end coordinate 335.

Finally, a radial third line 333 is drawn on the packaging from the end coordinate 335 inwards towards an edge of the central recess 130.

By cutting along the first line 331, the second line 332 and the third line 333, a piece 11 of the medical implant 100 of desired shape and size to fill the empty space in the natural meniscus can be cut out of the medical implant 100 in the medical product 300. A corresponding hole 340 is formed in the medical implant 100 and transparent packaging 310 (see Fig. 8D).

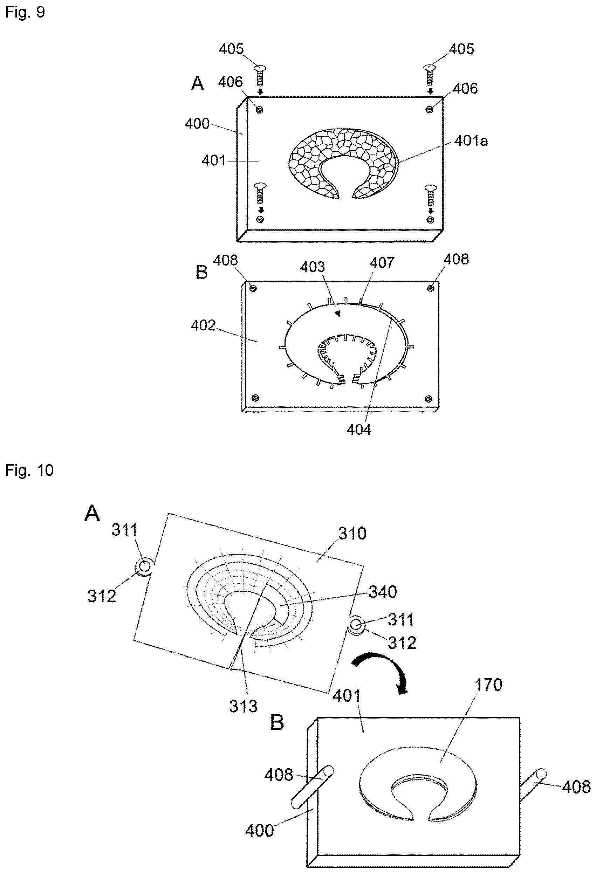

To assemble the cut-out piece 11 with a supporting body 170 to form a two-piece medical implant 100 as shown in Fig. 6B and 6C and described above (wherein the cut-out piece 11 is the replacement member 160), an alignment tool 400 as shown in Fig. 9 can be used.

The alignment tool 400 comprises a working surface 401, wherein a partial surface of the working surface 401 having the crescent moon shape of the medical implant 100 is particularly covered with a special smooth or spongy material which facilities the use of a felting needle 210 on the working surface 401. The alignment tool 400 further comprises a holder 402 comprising a cutout 403 resembling the crescent moon shape of the medical implant 100. The part comprising the working surface 401 comprises four bores 406 with an inner thread and the holder 402 comprise corresponding through-holes 408 which can be aligned with the bores 406 when the holder 402 is placed on the working surface 401. The holder 402 can then be fixed on the working surface 401 using fixing members 405, such as screws, which are inserted into the bores 406 and through-holes 408.

Notches 407 are arranged around an edge 404 of the cutout 403 of the holder 402, the notches 407 extending in the radial direction or the circumferential direction. These notches 407 are designed to guide a scalpel during cutting the medical implant 100 into a desired shape, e.g. along the first line 331, the second line 332 and the third line 333 indicated in Fig. 8B-D, which facilitates the cutting procedure.

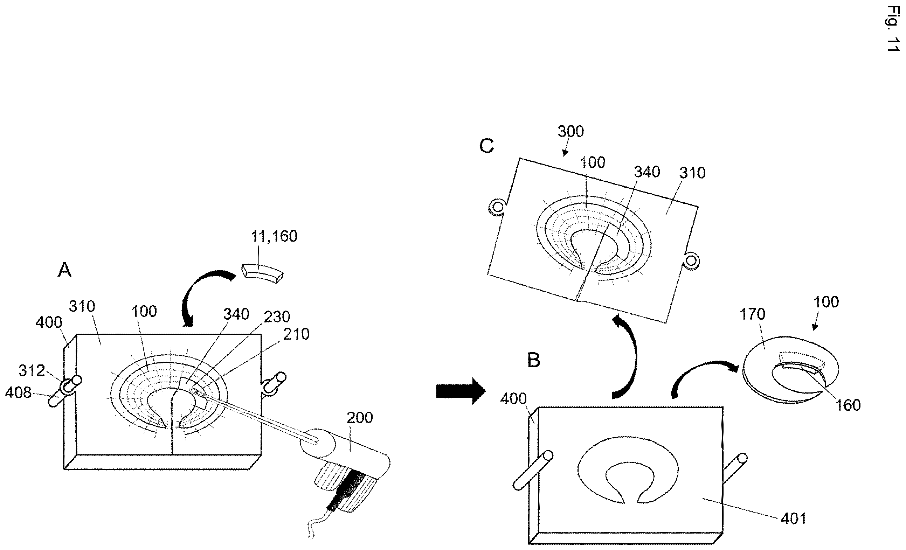

Fig. 10 and 11 show a method, by which a two-part medical implant 100, such as the one shown in Fig. 6B and 6C can be assembled from a supporting body 170 and a replacement member 160 using the previously described medical product 300 and the alignment tool 400.

First, the supporting body 170 is placed on the working surface 401 of the alignment tool 400, particularly on the spongy material 401a (Fig. 10B).

Subsequently, as shown in Fig. 10A and 10B, after cutting out the piece 11 from the medical product as illustrated in Fig. 8 and described above, the remaining medical product 300 comprising the hole 340 and a cut 313 leading from a lower edge of the packaging 310 to the hole 340 is placed on the working surface 401, and the outline marking 321 of the packaging 310 is aligned with the supporting body 170. The packaging 310 may be aligned and held in place by two pins 408 arranged on opposite sides of the working surface 401 which insert into corresponding through-holes 311 of the packaging. In the example depicted in Fig. 10, the through-holes 311 are arranged in latches 312.

Next, as illustrated in Fig. 11A, the piece 11 constituting the replacement member 160 is placed in the hole 340, and the replacement member 160 is connected to the supporting body 170 by felting, using the felting instrument 200.

Finally, the medical product 300 comprising the hole 340 and the finished medical implant 100 for partial meniscus replacement is removed from the alignment tool 400 (Fig. 11B and 11C).

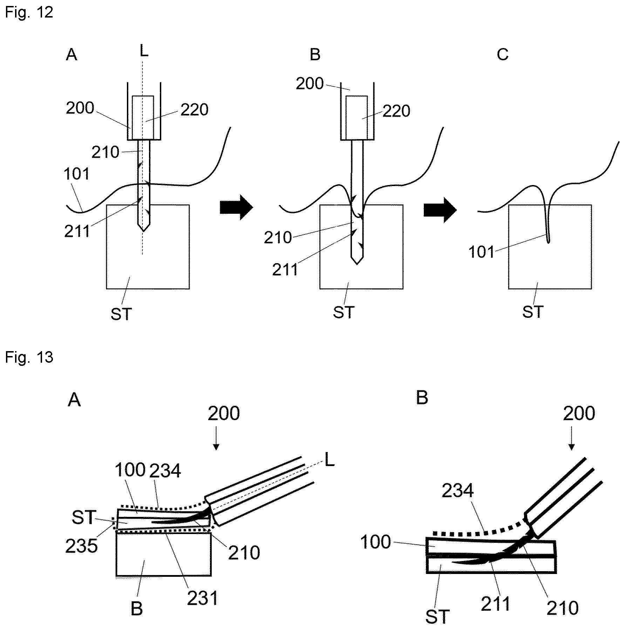

Fig. 12 shows a method of connecting fibers 101 of a felt material F to biological soft tissue ST by a surgical felting instrument 200, which can be used to connect the medical implant 100 according to the invention to soft tissue SF, such as a lateral or medial meniscus.

As shown in Fig. 12A-C, the barbs 211 on the felting needle 210 of the felting instrument 200 pull and push fibers 101 into the soft tissue ST when the felting needle is moved back and forth along the longitudinal axis L in the soft tissue ST driven by the drive 220.

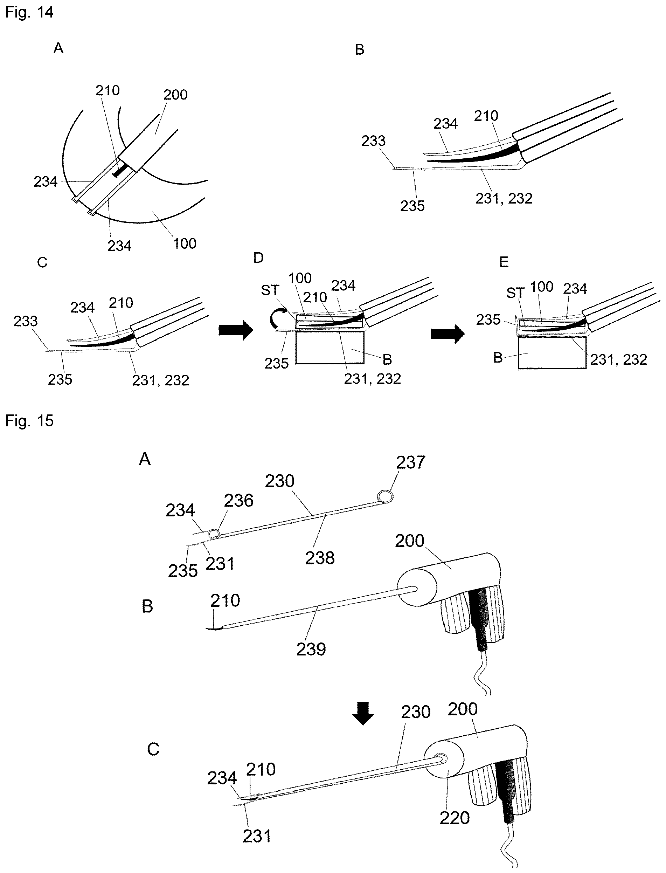

Fig. 13 and 14 show a specific embodiment of the felting instrument 200 according to the invention as it is used to connect a medical implant 100 comprising a felt material F to biological soft tissue ST arranged on a bone B. The soft tissue ST may be e.g. a natural meniscus, and the medical implant may be a meniscus implant formed according to the present invention.

The felting instrument 200 comprises a felting needle 210 comprising at least one barb 211 and a drive 220 for moving the felting needle 210 back and forth along a longitudinal axis L.

In the depicted embodiment, the felting needle 210 is curved, such that the medical implant 100 can be conveniently connected to the soft tissue ST without touching the bone B.

In addition, as shown in Fig. 13A and 14 A-E, the felting instrument 200 comprises a supporting member 230 comprising a lower member 231 configured to be advanced below the soft tissue ST or partially through the soft tissue ST to hold the soft tissue ST in place during felting. The supporting member 230 further comprises an upper member 234 configured to be placed on top of the medical implant 100 to apply force on the medical implant 100 and the soft tissue ST to ensure that their relative position does not change during felting. The lower member comprises a flippable end section 235 that can be flipped up towards the upper member 234 to support the medical implant 100 from the lateral side opposite the felting needle 210, and particularly form a cage-like compartment around the medical implant 100 and the soft tissue ST.

The flipping of the end section 235 is depicted in more detail in Fig. 14C-E

Alternatively, as depicted in Fig. 13B, the supporting member 230 may consist only of the upper member 234.

The lower member 231 may be formed as a needle 232 comprising a sharp tip 233, such that the lower member 231 can penetrate into and through the soft tissue ST, which improves fixation of the soft tissue ST.

Fig. 15 shows additional details of a felting instrument 200 (Fig. 15A) comprising a curved felting needle 210 comprising barbs 211 (not shown, see Fig. 12), and a supporting member 230 formed as a attachable and removable part (Fig. 15B). Fig. 15C shows the assembled felting instrument 200 including the supporting member 230.

The supporting member 230 comprises at least one lower part 231 with a flippable end section 235 and at least one upper part 234, wherein the lower part 231 and the upper part 234 are attached to a first ring 236. A connecting rod 238 connects the first ring 236 to a second ring 237 which may be attached to the felting instrument 200.

The connecting rod 238 and the rings 236, 237 are arranged such that a shaft 239, in which the felting needle 210 moves, is spaced apart from the connecting rod 238 and extends through the first ring 236 and the second ring 237. Thereby, the felting needle 210 cannot touch the supporting member 230 avoiding damage to the components of the felting instrument 200.

**List of reference signs**

| | |
|---|---|
| 10 | Sheet |
| 11 | Piece |
| 12 | Scissors |
| 100 | Medical implant |
| 101 | Fiber |
| 102 | Outside surface |
| 103 | Top surface |
| 104 | Rim surface |
| 111,112,113,114 | Zone |
| 121 | First end |
| 122 | Central section |
| 123 | Second end |
| 130 | Central recess |
| 131 | Opening |
| 132 | Edge |
| 140,141,142,143,144 | Layer |
| 140 | Bottom layer |
| 141 | Top layer |
| 142,143 | Intermediate layer |
| 144 | First cover layer |
| 145 | Second cover layer |
| 150 | Strip |
| 160 | Replacement member |
| 170 | Supporting body |
| 171 | Bottom surface |
| 200 | Felting instrument |
| 210 | Felting needle |
| 211 | Barb |
| 212 | Tip |
| 220 | Drive |
| 230 | Supporting member |
| 231 | Lower member |
| 232 | Needle |
| 233 | Tip |
| 234 | Upper member |
| 235 | End section |
| 236 | First ring |
| 237 | Second ring |
| 238 | Connecting part |
| 239 | Shaft |
| 300 | Medical product |
| 310 | Transparent packaging |
| 311 | Through-hole |
| 312 | Latch |
| 313 | Cut |
| 320 | Markings |
| 321 | Outline marking |
| 322 | Radial scale marking |
| 323 | Circumferential scale marking |
| 331 | First line |
| 332 | Second line |
| 333 | Third line |
| 334 | Start coordinate |
| 335 | End coordinate |
| 340 | Hole |
| 400 | Alignment tool |
| 401 | Working surface |
| 401a | Spongy material |
| 402 | Holder |
| 403 | Cutout |
| 404 | Edge |
| 405 | Fixing means |
| 406 | Bore |
| 407 | Notch |
| 408 | Through-hole |
| A1 | First axis |
| B | Bone |
| C | Circumferential direction |
| CL | Curved line |
| F | Felt material |
| L | Longitudinal axis |
| P | Plane |
| R | Radial direction |
| ST | Soft Tissue |

## Claims

1. A medical implant (100), particularly for meniscus repair or replacement, wherein the medical implant (100) extends along a plane (P) perpendicular to a first axis (A1), and wherein the medical implant (100) comprises a felt material (F) comprising a plurality of fibers (101), **characterized in that** a density of said fibers (101) varies along said plane (P) resulting in a varying compressive strength along said first axis (A1), and/or a percentage of said fibers (101) aligned in a circumferential direction (C) in respect of said first axis (A1) differs along said plane (P) resulting in a varying tensile strength of the medical implant (100) along said plane (P).

2. The medical implant (100) according to claim 1, wherein said medical implant (100) comprises an outside surface (102), wherein said density of said fibers (101) increases in a radial direction (R) in respect of the first axis (A1) towards the outside surface (102), particularly such that said compressive strength increases in the radial direction (R) towards the outside surface (102), and/or said percentage of said fibers (101) aligned in said circumferential direction (C) increases in the radial direction (R) towards the outside surface (102), particularly such that said tensile strength of the medical implant (100) increases in said radial direction (R) towards said outside surface (102).

3. The medical implant (100) according to claim 1 or 2, wherein said medical implant comprises a plurality of zones (110, 111, 112, 113), wherein said density of said fibers (101) and/or said percentage of said fibers (101) aligned in the circumferential direction (C) varies between the zones (110, 111, 112, 113), wherein particularly said zones (110, 111, 112, 113) are arranged along said radial direction (R).

4. The medical implant (100) according to any one of the preceding claims, wherein said medical implant extends from a first end (121) via a central section (122) towards a second end (123) along a curved line (CL) extending in the circumferential direction (C), wherein the medical implant (100) comprises a central recess (130), around which the curved line (CL) extends.

5. The medical implant (100) according to any one of the preceding claims, wherein said medical implant is formed from a plurality of layers (140, 141, 142, 143, 144) of said felt material (F), wherein the layers (140, 141, 142, 143, 144) are stacked in a direction parallel to the first axis (A1), and wherein said layers (140, 141, 142, 143, 144) are connected by felting.

6. The medical implant (100) according to claim 5 when referring back to claim 4, wherein said layers (140, 141, 142, 142, 144) comprise a bottom layer (140) and a top layer (141), wherein an arc length of the bottom layer extending along the curved line (CL) is greater than an arc length of the top layer (141) extending along the curved line (CL), such that a thickness of the medical implant (100) measured parallel to the first axis (A1) increases from the first end (121) towards the central section (122) and/or from the second end (123) towards the central section (122), wherein particularly said layers (140, 141, 142, 144) comprise at least one intermediate layer (142, 143) between the bottom layer (140) and the top layer (141), wherein an arc length of the at least one intermediate layer (142, 143) is between the arc length of the bottom layer (140) and the arc length of the top layer (141), wherein more particularly the medical implant (100) further comprises a first cover layer (144) covering the bottom layer (140), the intermediate layers (142, 143) and the top layer (141) on a top surface (103), particularly from the first end (121) towards the second end (122) and/or the medical implant (100) further comprises a second cover layer (145) covering said bottom layer (140), said intermediate layers (142, 143) and said top layer (141) on a rim surface (104).

7. The medical implant (100) according to claim 6, wherein said medical implant (100) comprises a strip (150) comprising said felt material (F), wherein said strip (150) is arranged in said circumferential direction (C) in respect of the first axis (A1) along an outside surface (102) of the medical implant (100) between the bottom layer (140) and the top layer (141), wherein particularly said strip (150) extends along said curved line (CL), wherein more particularly said strip (150) has an arc length along the curved line (CL) which is shorter than the arc length of the bottom layer (140) and shorter than the arc length of the top layer (141).

8. The medical implant (100) according to claim 4 or any one of the claims 5 to 7, when referring back to claim 4, wherein said medical implant (100) comprises a replacement member (160) configured to replace a part of a soft biological tissue (ST), particularly a meniscus, and a supporting body (170) connected to the replacement member (160), wherein the supporting body (170) extends from the first end (121) via the central section (122) to the second end (123) along the curved line (CL) in the circumferential direction (C), and wherein the replacement member (160) is arranged on a bottom surface (171) of the supporting body (170), wherein the replacement member (160) covers a partial segment of said bottom surface (171), wherein particularly said replacement member (160) has a thickness measured parallel to the first axis (A1) which is greater than a thickness of the supporting body (170) measured parallel to the first axis (A1).

9. A method of manufacturing a medical implant (100) according to one of the claims 1 to 8, wherein at least one sheet (10) comprising a felt material (F) is provided, the sheet (10) extending along a plane (P) perpendicular to a first axis (A1), wherein the felt material (F) comprises a plurality of fibers (101), and wherein a felting needle (210) comprising at least one barb (211) is repeatedly advanced through the sheet (10) at specified locations to increase the density of the fibers (101) such that a density of said fibers (101) varies along said plane (P) such that the compressive strength of the sheet (10) measured parallel to the first axis (A1) is increased at the specified locations and/or to increase the percentage of fibers (101) aligned in a circumferential direction (C) in respect of the first axis (A1) such that a percentage of said fibers (101) aligned in a circumferential direction (C) in respect of said first axis (A1) differs along said plane (P) such that the tensile strength of the sheet (10) measured parallel to the plane (P) is increased at the specified locations.

10. A medical product (300) comprising a medical implant (100) according to one of the claims 1 to 8, and a transparent packaging (310), wherein the packaging (310) comprises printed markings (320) comprising an outline marking (321) of the shape of the medical implant (100), a radial scale marking (322) and a circumferential scale marking (323), wherein the medical implant (100) is arranged in the packaging (310), such that the outline marking (321) is aligned with the medical implant (100).

11. A kit including a medical implant (100) according to one of the claims 1 to 8 and/or a medical product (300) according to claim 10 respectively with an alignment tool (400) comprising a working surface (401) configured to receive the medical product according to claim 10 and/or the medical implant (100) according to one of the claims 1 to 8, and a holder (402) configured to be placed on the working surface (401), wherein the holder (402) comprises a cutout (403) having a size and shape of the medical implant (100), particularly such that the outline marking (321) and/or the medical implant (100) can be aligned with the cutout (403), wherein particularly the working surface (401) is covered by a spongy material (401a).

12. A method for customizing a medical implant, particularly according to one of the claims 1 to 8, wherein a medical product (300) according to claim 10 is provided, and wherein a desired shape of the medical implant (100) is marked on the transparent packaging (310) of the medical product (300) using the radial scale marking (322) and the circumferential scale marking (323, and wherein a piece (11) is cut out of the medical implant (100) and the packaging (310) using the marked desired shape.

13. A kit including a medical implant (100) according to one of the claims 1 to 8 and/or a medical product (300) according to claim 10 respectively with a felting instrument (200) comprising a felting needle (210) comprising at least one barb (211), wherein the felting instrument (200) comprises a drive (220) configured to move the felting needle (210) back and forth along a longitudinal axis (L).

14. The kit according to claim 13, wherein the felting needle (210) is curved.

15. The kit setaccording to claim 13 or 14, wherein the felting instrument (200) comprises a supporting member (230) configured to hold a medical implant (100) according to one of the claims 1 to 8 in place on a biological soft tissue (ST), particularly a meniscus, such that the felting needle (210) of the felting instrument (200) can be advanced through the medical implant (100) and the soft tissue (ST) to connect the medical implant (100) to the biological soft tissue (ST), particularly wherein the supporting member (230) comprises a lower member (231) configured to be arranged below the soft biological tissue (ST) and/or through the soft biological tissue (ST), and wherein the supporting member (230) comprises an upper member (234) configured to be arranged on the medical implant (100), particularly to apply pressure on the medical implant (100), more particularly wherein said lower member (231) comprises an end section (235) that is flippable to connect the end section (235) to the upper member (234).

## Patentansprüche

1. Medizinisches Implantat (100), insbesondere zur Meniskusreparatur oder zum Meniskusersatz, wobei sich das medizinische Implantat (100) entlang einer Ebene (P) senkrecht zu einer ersten Achse (A1) erstreckt und wobei das medizinische Implantat (100) ein Filzmaterial (F) mit mehreren Fasern (101) aufweist, **dadurch gekennzeichnet, dass** eine Dichte der Fasern (101) entlang der Ebene (P) variiert, was zu einer variierenden Druckfestigkeit entlang der ersten Achse (A1) führt, und/oder sich ein Prozentsatz der Fasern (101), die in einer Umfangsrichtung (C) im Hinblick auf die erste Achse (A1) ausgerichtet sind, entlang der Ebene (P) unterscheidet, was zu einer variierenden Zugfestigkeit des medizinischen Implantats (100) entlang der Ebene (P) führt.

2. Medizinisches Implantat (100) nach Anspruch 1, wobei das medizinische Implantat (100) eine Außenfläche (102) aufweist, wobei die Dichte der Fasern (101) in einer Radialrichtung (R) im Hinblick auf die erste Achse (A1) hin zur Außenfläche (102) zunimmt, insbesondere so, dass die Druckfestigkeit in der Radialrichtung (R) hin zur Außenfläche (102) zunimmt und/oder der Prozentsatz der Fasern (101), die in der Umfangsrichtung (C) ausgerichtet sind, in der Radialrichtung (R) hin zur Außenfläche (102) zunimmt, insbesondere so, dass die Zugfestigkeit des medizinischen Implantats (100) in der Radialrichtung (R) hin zur Außenfläche (102) zunimmt.

3. Medizinisches Implantat (100) nach Anspruch 1 oder 2, wobei das medizinische Implantat mehrere Zonen (110, 111, 112, 113) aufweist, wobei die Dichte der Fasern (101) und/oder der Prozentsatz der Fasern (101), die in der Umfangsrichtung (C) ausgerichtet sind, zwischen den Zonen (110, 111, 112, 113) variiert, wobei die Zonen (110, 111, 112, 113) insbesondere entlang der Radialrichtung (R) angeordnet sind.

4. Medizinisches Implantat (100) nach einem der vorstehenden Ansprüche, wobei sich das medizinische Implantat von einem ersten Ende (121) über ein Mittelteilstück (122) hin zu einem zweiten Ende (123) entlang einer sich in der Umfangsrichtung (C) erstreckenden gekrümmten Linie (CL) erstreckt, wobei das medizinische Implantat (100) eine Mittelaussparung (130) aufweist, um die sich die gekrümmte Linie (CL) erstreckt.

5. Medizinisches Implantat (100) nach einem der vorstehenden Ansprüche, wobei das medizinische Implantat aus mehreren Schichten (140, 141, 142, 143, 144) des Filzmaterials (F) gebildet ist, wobei die Schichten (140, 141, 142, 143, 144) in einer Richtung parallel zur ersten Achse (A1) gestapelt sind und wobei die Schichten (140, 141, 142, 143, 144) durch Filzen verbunden sind.

6. Medizinisches Implantat (100) nach Ansprüchen 4 und 5 Anspruch 4, wobei die Schichten (140, 141, 142, 142, 144) eine Bodenschicht (140) und eine Kopfschicht (141) aufweisen, wobei eine Bogenlänge der sich entlang der gekrümmten Linie (CL) erstreckenden Bodenschicht größer ist als eine Bogenlänge der sich entlang der gekrümmten Linie (CL) erstreckenden Kopfschicht (141), so dass eine Dicke des medizinischen Implantats (100) in der Messung parallel zur ersten Achse (A1) vom ersten Ende (121) hin zum Mittelteilstück (122) und/oder vom zweiten Ende (123) hin zum Mittelteilstück (122) zunimmt, wobei die Schichten (140, 141, 142, 144) insbesondere mindestens eine Zwischenschicht (142, 143) zwischen der Bodenschicht (140) und der Kopfschicht (141) aufweisen, wobei eine Bogenlänge der mindestens einen Zwischenschicht (142, 143) zwischen der Bogenlänge der Bodenschicht (140) und der Bogenlänge der Kopfschicht (141) liegt, wobei das medizinische Implantat (100) insbesondere ferner eine erste Deckschicht (144) aufweist, die die Bodenschicht (140), die Zwischenschichten (142, 143) und die Kopfschicht (141) auf einer Kopffläche (103) insbesondere vom ersten Ende (121) hin zum zweiten Ende (122) abdeckt, und/oder das medizinische Implantat (100) ferner eine zweite Deckschicht (145) aufweist, die die Bodenschicht (140), die Zwischenschichten (142, 143) und die Kopfschicht (141) auf einer Randfläche (104) abdeckt.

7. Medizinisches Implantat (100) nach Anspruch 6, wobei das medizinische Implantat (100) einen Streifen (150) mit dem Filzmaterial (F) aufweist, wobei der Streifen (150) in der Umfangsrichtung (C) im Hinblick auf die erste Achse (A1) entlang einer Außenfläche (102) des medizinischen Implantats (100) zwischen der Bodenschicht (140) und der Kopfschicht (141) angeordnet ist, wobei sich der Streifen (150) insbesondere entlang der gekrümmten Linie (CL) erstreckt, wobei der Streifen (150) insbesondere eine Bogenlänge entlang der gekrümmten Linie (CL) hat, die kürzer als die Bogenlänge der Bodenschicht (140) und kürzer als die Bogenlänge der Kopfschicht (141) ist.

8. Medizinisches Implantat (100) nach Anspruch 4 oder einem der Ansprüche 5 bis 7 mit erneutem Bezug auf Anspruch 4, wobei das medizinische Implantat (100) ein Ersatzelement (160), das dazu konfiguriert ist, einen Teil eines biologischen Weichgewebes (ST), insbesondere einen Meniskus zu ersetzen, und einen mit dem Ersatzelement (160) verbundenen Stützkörper (170) aufweist, wobei sich der Stützkörper (170) vom ersten Ende (121) über das Mittelteilstück (122) zum zweiten Ende (123) entlang der gekrümmten Linie (CL) in der Umfangsrichtung (C) erstreckt und wobei das Ersatzelement (160) auf einer Bodenfläche (171) des Stützkörpers (170) angeordnet ist, wobei das Ersatzelement (160) ein Teilsegment der Bodenfläche (171) abdeckt, wobei das Ersatzelement (160) insbesondere eine Dicke in der Messung parallel zur ersten Achse (A1) hat, die größer ist als eine Dicke des Stützkörpers (170) in der Messung parallel zur ersten Achse (A1).

9. Verfahren zur Herstellung eines medizinischen Implantats (100) nach einem der Ansprüche 1 bis 8, wobei mindestens eine Matte (10) mit einem Filzmaterial (F) bereitgestellt wird, wobei sich die Matte (10) entlang einer Ebene (P) senkrecht zu einer ersten Achse (A1) erstreckt, wobei das Filzmaterial (F) mehrere Fasern (101) aufweist und wobei eine Filznadel (210) mit mindestens einem Widerhaken (211) durch die Matte (10) hindurch an festgelegten Stellen wiederholt vorbewegt wird, um die Dichte der Fasern (101) so zu erhöhen, dass eine Dichte der Fasern (101) entlang der Ebene (P) variiert, so dass die Druckfestigkeit der Matte (10) in der Messung parallel zur ersten Achse (A1) an den festgelegten Stellen erhöht wird, und/oder um den Prozentsatz von Fasern (101), die in einer Umfangsrichtung (C) im Hinblick auf die erste Achse (A1) ausgerichtet sind, so zu erhöhen, dass sich ein Prozentsatz der Fasern (101), die in einer Umfangsrichtung (C) im Hinblick auf die erste Achse (A1) ausgerichtet sind, entlang der Ebene (P) unterscheidet, so dass die Zugfestigkeit der Matte (10) in der Messung parallel zur Ebene (P) an den festgelegten Stellen erhöht wird.

10. Medizinprodukt (300) mit einem medizinischen Implantat (100) nach einem der Ansprüche 1 bis 8 und einer transparenten Verpackung (310), wobei die Verpackung (310) aufgedruckte Markierungen (320) aufweist, darunter eine Konturmarkierung (321) der Form des medizinischen Implantats (100), eine Radialskalenmarkierung (322) und eine Umfangsskalenmarkierung (323), wobei das medizinische Implantat (100) in der Verpackung (310) so angeordnet ist, dass die Konturmarkierung (321) zum medizinischen Implantat (100) ausgerichtet ist.

11. Kit, das ein medizinisches Implantat (100) nach einem der Ansprüche 1 bis 8 und/oder ein Medizinprodukt (300) nach Anspruch 10 aufweist, jeweils mit einem Ausrichtwerkzeug (400) mit einer Arbeitsfläche (401), die so konfiguriert ist, dass sie das Medizinprodukt nach Anspruch 10 und/oder das medizinische Implantat (100) nach einem der Ansprüche 1 bis 8 aufnimmt, und einem Halter (402), der so konfiguriert ist, dass er auf der Arbeitsfläche (401) platziert wird, wobei der Halter (402) einen Ausschnitt (403) mit einer Größe und Form des medizinischen Implantats (100) aufweist, insbesondere so, dass die Konturmarkierung (321) und/oder das medizinische Implantat (100) zum Ausschnitt (403) ausgerichtet werden können, wobei die Arbeitsfläche (401) insbesondere durch ein schwammiges Material (401a) abgedeckt ist.

12. Verfahren zur Anpassung eines medizinischen Implantats, insbesondere nach einem der Ansprüche 1 bis 8, wobei ein Medizinprodukt (300) nach Anspruch 10 bereitgestellt wird und wobei eine gewünschte Form des medizinischen Implantats (100) auf der transparenten Verpackung (310) des Medizinprodukts (300) mit Hilfe der Radialskalenmarkierung (322) und der Umfangsskalenmarkierung (323) markiert wird und wobei ein Stück (11) aus dem medizinischen Implantat (100) und der Verpackung (310) mit Hilfe der markierten gewünschten Form ausgeschnitten wird.

13. Kit, das ein medizinisches Implantat (100) nach einem der Ansprüche 1 bis 8 und/oder ein Medizinprodukt (300) nach Anspruch 10 aufweist, jeweils mit einem Filzinstrument (200), das eine Filznadel (210) mit mindestens einem Widerhaken (211) aufweist, wobei das Filzinstrument (200) einen Antrieb (220) aufweist, der dazu konfiguriert ist, die Filznadel (210) entlang einer Längsachse (L) hin und her zu bewegen.

14. Kit nach Anspruch 13, wobei die Filznadel (210) gekrümmt ist.

15. Kit nach Anspruch 13 oder 14, wobei das Filzinstrument (200) ein Stützelement (230) aufweist, das dazu konfiguriert ist, ein medizinisches Implantat (100) nach einem der Ansprüche 1 bis 8 an einem Ort auf einem biologischen Weichgewebe (ST), insbesondere einem Meniskus, so zu halten, dass die Filznadel (210) des Filzinstruments (200) durch das medizinische Implantat (100) und das Weichgewebe (ST) hindurch vorbewegt werden kann, um das medizinische Implantat (100) mit dem biologischen Weichgewebe (ST) zu verbinden, wobei das Stützelement (230) insbesondere ein unteres Element (231) aufweist, das so konfiguriert ist, dass es unter dem biologischen Weichgewebe (ST) und/oder durch das biologische Weichgewebe (ST) hindurch angeordnet wird, und wobei das Stützelement (230) ein oberes Element (234) aufweist, das so konfiguriert ist, dass es auf dem medizinischen Implantat (100) angeordnet wird, um insbesondere Druck auf das medizinische Implantat (100) auszuüben, wobei das untere Element (231) insbesondere ein Endteilstück (235) aufweist, das umklappbar ist, um das Endteilstück (235) mit dem oberen Element (234) zu verbinden.

## Revendications

1. Implant médical (100), en particulier pour la réparation ou le remplacement du ménisque, dans lequel l'implant médical (100) s'étend le long d'un plan (P) perpendiculaire à un premier axe (A1), et dans lequel l'implant médical (100) comprend un matériau feutré (F) comprenant une pluralité de fibres (101), **caractérisé en ce que** la densité desdites fibres (101) varie le long dudit plan (P), ce qui entraîne une résistance à la compression variable le long dudit premier axe (A1), et/ou un pourcentage desdites fibres (101) alignées dans une direction circonférentielle (C) par rapport audit premier axe (A1) diffère le long dudit plan (P), ce qui entraîne une résistance à la traction variable de l'implant médical (100) le long dudit plan (P).

2. Implant médical (100) selon la revendication 1, dans lequel ledit implant médical (100) comprend une surface extérieure (102), dans lequel ladite densité desdites fibres (101) augmente dans une direction radiale (R) par rapport au premier axe (A1) vers la surface extérieure (102), en particulier de sorte que ladite résistance à la compression augmente dans la direction radiale (R) vers la surface extérieure (102), et/ou ledit pourcentage desdites fibres (101) alignées dans ladite direction circonférentielle (C) augmente dans la direction radiale (R) vers la surface extérieure (102), en particulier de sorte que ladite résistance à la traction de l'implant médical (100) augmente dans ladite direction radiale (R) vers ladite surface extérieure (102).

3. Implant médical (100) selon la revendication 1 ou 2, dans lequel ledit implant médical comprend une pluralité de zones (110, 111, 112, 113), dans lequel ladite densité desdites fibres (101) et/ou ledit pourcentage desdites fibres (101) alignées dans la direction circonférentielle (C) varie entre les zones (110, 111, 112, 113), dans lequel en particulier lesdites zones (110, 111, 112, 113) sont agencées le long de ladite direction radiale (R).

4. Implant médical (100) selon l'une quelconque des revendications précédentes, dans lequel ledit implant médical s'étend depuis une première extrémité (121) par l'intermédiaire d'une section centrale (122) vers une deuxième extrémité (123) le long d'une ligne courbe (CL) s'étendant dans la direction circonférentielle (C), dans lequel l'implant médical (100) comprend un évidement central (130) autour duquel s'étend la ligne courbe (CL).

5. Implant médical (100) selon l'une quelconque des revendications précédentes, dans lequel ledit implant médical est formé à partir d'une pluralité de couches (140, 141, 142, 143, 144) dudit matériau feutré (F), dans lequel les couches (140, 141, 142, 143, 144) sont empilées dans une direction parallèle au premier axe (A1), et dans lequel lesdites couches (140, 141, 142, 143, 144) sont reliées par feutrage.

6. Implant médical (100) selon la revendication 5, lorsqu'elle fait référence à la revendication 4, dans lequel lesdites couches (140, 141, 142, 142, 144) comprennent une couche inférieure (140) et une couche supérieure (141), dans lequel une longueur d'arc de la couche inférieure s'étendant le long de la ligne courbe (CL) est supérieure à une longueur d'arc de la couche supérieure (141) s'étendant le long de la ligne courbe (CL), de sorte qu'une épaisseur de l'implant médical (100) mesurée parallèlement au premier axe (A1) augmente depuis la première extrémité (121) vers la section centrale (122) et/ou depuis la deuxième extrémité (123) vers la section centrale (122), dans lequel en particulier lesdites couches (140, 141, 142, 144) comprennent au moins une couche intermédiaire (142, 143) entre la couche inférieure (140) et la couche supérieure (141), dans lequel une longueur d'arc de l'au moins une couche intermédiaire (142, 143) est comprise entre la longueur d'arc de la couche inférieure (140) et la longueur d'arc de la couche supérieure (141), dans lequel, plus particulièrement, l'implant médical (100) comprend en outre une première couche de recouvrement (144) recouvrant la couche inférieure (140), les couches intermédiaires (142, 143) et la couche supérieure (141) sur une surface supérieure (103), en particulier depuis la première extrémité (121) vers la deuxième extrémité (122), et/ou l'implant médical (100) comprend en outre une deuxième couche de recouvrement (145) recouvrant ladite couche inférieure (140), lesdites couches intermédiaires (142, 143) et ladite couche supérieure (141) sur une surface de rebord (104).

7. Implant médical (100) selon la revendication 6, dans lequel ledit implant médical (100) comprend une bande (150) comprenant ledit matériau feutré (F), dans lequel ladite bande (150) est agencée dans ladite direction circonférentielle (C) par rapport au premier axe (A1) le long d'une surface extérieure (102) de l'implant médical (100) entre la couche inférieure (140) et la couche supérieure (141), dans lequel en particulier ladite bande (150) s'étend le long de ladite ligne courbe (CL), dans lequel plus particulièrement ladite bande (150) a une longueur d'arc le long de la ligne courbe (CL) qui est plus courte que la longueur d'arc de la couche inférieure (140) et plus courte que la longueur d'arc de la couche supérieure (141).

8. Implant médical (100) selon la revendication 4, ou selon l'une quelconque des revendications 5 à 7, lorsqu'elle fait référence à la revendication 4, dans lequel ledit implant médical (100) comprend un élément de remplacement (160) configuré pour remplacer une partie d'un tissu biologique mou (ST), en particulier un ménisque, et un corps de support (170) relié à l'élément de remplacement (160), dans lequel le corps de support (170) s'étend de la première extrémité (121) par l'intermédiaire de la section centrale (122) à la deuxième extrémité (123) le long de la ligne courbe (CL) dans la direction circonférentielle (C), et dans lequel l'élément de remplacement (160) est agencé sur une surface inférieure (171) du corps de support (170), dans lequel l'élément de remplacement (160) recouvre un segment partiel de ladite surface inférieure (171), dans lequel en particulier ledit élément de remplacement (160) a une épaisseur mesurée parallèlement au premier axe (A1) qui est supérieure à une épaisseur du corps de support (170) mesurée parallèlement au premier axe (A1).

9. Procédé de fabrication d'un implant médical (100) selon l'une quelconque des revendications 1 à 8, dans lequel au moins une feuille (10) comprenant un matériau feutré (F) est fournie, la feuille (10) s'étendant le long d'un plan (P) perpendiculaire à un premier axe (A1), dans lequel le matériau feutré (F) comprend une pluralité de fibres (101), et dans lequel une aiguille de feutrage (210) comprenant au moins un ardillon (211) est avancée de manière répétée à travers la feuille (10) à des emplacements spécifiés afin d'augmenter une densité des fibres (101), de sorte que la densité desdites fibres (101) varie le long dudit plan (P), de sorte que la résistance à la compression de la feuille (10), mesurée parallèlement au premier axe (A1), est augmentée aux emplacements spécifiés, et/ou afin d'augmenter un pourcentage de fibres (101) alignées dans une direction circonférentielle (C) par rapport au premier axe (A1), de sorte que le pourcentage desdites fibres (101) alignées dans une direction circonférentielle (C) par rapport audit premier axe (A1) diffère le long dudit plan (P), de sorte que la résistance à la traction de la feuille (10) mesurée parallèlement au plan (P) est augmentée aux emplacements spécifiés.

10. Produit médical (300) comprenant un implant médical (100) selon l'une quelconque des revendications 1 à 8, et un emballage transparent (310), dans lequel l'emballage (310) comprend des marquages imprimés (320) comprenant un marquage de contour (321) de la forme de l'implant médical (100), un marquage d'échelle radiale (322) et un marquage d'échelle circonférentielle (323), dans lequel l'implant médical (100) est agencé dans l'emballage (310) de sorte que le marquage de contour (321) est aligné avec l'implant médical (100).

11. Kit comprenant un implant médical (100) selon l'une quelconque des revendications 1 à 8 et/ou un produit médical (300) selon la revendication 10, respectivement avec un outil d'alignement (400) comprenant une surface de travail (401) configurée pour recevoir le produit médical selon la revendication 10 et/ou l'implant médical (100) selon l'une quelconque des revendications 1 à 8, et un support (402) configuré pour être placé sur la surface de travail (401), dans lequel le support (402) comprend une découpe (403) ayant une taille et une forme de l'implant médical (100), en particulier de sorte que le marquage de contour (321) et/ou l'implant médical (100) peut/peuvent être aligné(s) avec la découpe (403), dans lequel, en particulier, la surface de travail (401) est recouverte d'un matériau spongieux (401a).

12. Procédé de personnalisation d'un implant médical, en particulier selon l'une quelconque des revendications 1 à 8, dans lequel un produit médical (300) selon la revendication 10 est fourni, et dans lequel une forme souhaitée de l'implant médical (100) est marquée sur l'emballage transparent (310) du produit médical (300) à l'aide du marquage d'échelle radiale (322) et du marquage d'échelle circonférentielle (323), et dans lequel un morceau (11) est découpé dans l'implant médical (100) et l'emballage (310) à l'aide de la forme souhaitée marquée.

13. Kit comprenant un implant médical (100) selon l'une quelconque des revendications 1 à 8 et/ou un produit médical (300) selon la revendication 10, respectivement avec un instrument de feutrage (200) comprenant une aiguille de feutrage (210) comprenant au moins un ardillon (211), dans lequel l'instrument de feutrage (200) comprend un entraînement (220) configuré pour déplacer l'aiguille de feutrage (210) d'avant en arrière le long d'un axe longitudinal (L).

14. Kit selon la revendication 13, dans lequel l'aiguille de feutrage (210) est courbée.

15. Kit selon la revendication 13 ou 14, dans lequel l'instrument de feutrage (200) comprend un élément de support (230) configuré pour maintenir un implant médical (100) selon l'une quelconque des revendications 1 à 8 en place sur un tissu biologique mou (ST), en particulier un ménisque, de sorte que l'aiguille de feutrage (210) de l'instrument de feutrage (200) peut être avancée à travers l'implant médical (100) et le tissu biologique mou (ST) afin de relier l'implant médical (100) au tissu biologique mou (ST), en particulier dans lequel l'élément de support (230) comprend un élément inférieur (231) configuré pour être agencé sous le tissu biologique mou (ST) et/ou à travers le tissu biologique mou (ST), et dans lequel l'élément de support (230) comprend un élément supérieur (234) configuré pour être agencé sur l'implant médical (100), en particulier afin d'appliquer une pression sur l'implant médical (100), plus particulièrement dans lequel ledit élément inférieur (231) comprend une section d'extrémité (235) qui peut être rabattue pour relier la section d'extrémité (235) à l'élément supérieur (234).
